Europäisches Patentamt

European Patent Office

Office européen des brevets

(11) Publication number: **0 453 683 B1**

# EUROPEAN PATENT SPECIFICATION

(45) Date of publication of patent specification: **20.07.94**  (51) Int. Cl.5: **A61K 7/00**

(21) Application number: **90311713.3**

(22) Date of filing: **25.10.90**

(54) **Cosmetic capsules.**

(30) Priority: **26.09.90 US 588249**
**27.04.90 US 515286**

(43) Date of publication of application:
**30.10.91 Bulletin 91/44**

(45) Publication of the grant of the patent:
**20.07.94 Bulletin 94/29**

(84) Designated Contracting States:
**AT BE CH DE DK ES FR GB GR IT LI NL SE**

(56) References cited:
**EP-A- 0 186 502**
**FR-A- 2 556 940**
**US-A- 4 429 927**

**SOFW: SEIFEN, OLE, FETTE, WACHSE, vol. 116, no. 5, 19th March 1990, pages 161-163; D. STEPHAN: "Verkapselung von Wirkstoffen in Pharmazie und Kosmetik"**

**IDEM**

**SÖWF - SEIFEN, ÖLE, FETTE, WACHSE, vol. 113, no. 2, 19th February 1987, pages 67-71, Augsburg, DE; H. JAHN: "Kosmetik in Kapseln"**

(73) Proprietor: **UNILEVER PLC**
**Unilever House**
**Blackfriars**
**P.O. Box 68**
**London EC4P 4BO(GB)**

(84) Designated Contracting States:
**GB**

(73) Proprietor: **UNILEVER N.V.**
**Weena 455**
**NL-3013 AL Rotterdam(NL)**

(84) Designated Contracting States:
**BE CH DE DK ES FR GR IT LI NL SE AT**

(72) Inventor: **Spellman, Joseph Xavier**
**1 Silver Brook Road**
**Westport, Connecticut 06880(US)**
Inventor: **Beck, Marlene C.**
**301 East 64th Street**
**New York, New York 10021(US)**
Inventor: **Connell, Victoria**
**30 Christopher Street**
**New York, New York 10014(US)**

(74) Representative: **Elliott, Peter William et al**
**Unilever plc**
**Patent Division**
**Colworth House**
**Sharnbrook**
**Bedford MK44 1LQ (GB)**

**Description**

1. Field of the Invention

The invention concerns capsules containing unit doses of cosmetic compositions.

2. The Related Art

Cosmetics are generally packaged in relatively large containers in amounts that provide multiple doses. Bulk packaging has certain disadvantages. Once opened, the contents of a package become exposed to moisture and air. These forces can be quite detrimental to sensitive ingredients forming the cosmetic product. With bulk packaging, a manufacturer also cannot control individual dosage levels which are most effective and safe; the consumer is burdened with this responsibility. Invariably, usage will either be too high or too low.

Single or unit dose packages of various descriptions have been disclosed in the art. Capsules are one of the newest vehicles for delivering unit dosages of cosmetic products. Recently the Revlon Corporation introduced a product called Age-less®. The product is a composition of vitamin E, sunscreens and moisturizers which have been sealed into vitamin-like capsules. These elongated capsules are meant to be pierced and their contents squeezed onto the skin. Certain problems are, however, associated with the packaging vehicle. For instance, a sharp pointed instrument is necessary to pierce the capsule walls; this nay readily lead to injury. An opening mechanism that would avoid necessity for procuring any opening instrument would also be more convenient. Furthermore, there is danger in shaping cosmetic products to look like vitamins. Children, or even adults, may inadvertently mistake the product and ingest it.

La Prairie Corporation has recently introduced a cosmetic called Skin Caviar® which is a skin-care lotion contained in tiny egglike globes that are popped and rubbed onto the face. A problem with round packaging lies in the tendency for them to roll away. There is also no easy handle by which they may be gripped.

Seifen, Ole, Fette, Wachse, Vol 113, No. 2, p 67-71, describes cosmetic capsules which contain a cosmetic material, with the capsule walls being soft and made of gelatin. The capsules may be used for various purposes, including delivering benefit agents and perfumes in baths, skin oils, sun oils, water free creams, toothpastes, ethereal oils, eye shadows, and so on. Such capsules may have twist off nipples.

Seifen, Ole, Fette, Wachse, Vol 116, No. 5, p 161-163 describes production techniques for hard and soft gelatin capsules, including various different capsule forms. Anhydrous and water containing formulations are mentioned as suitable for encapsulation. "Twist off" forms of capsule are also mentioned.

FR 2556940 (Laboratoire Cantapharm Sarl) describes gelatin capsules with an integral capillary tube, which may be used for delivering cosmetic agents to the body, in particular to the eyes and face.

Accordingly, it is an object of the present invention to provide a cosmetic product delivered in a capsule which avoids many of the problems associated with the known art.

A more specific object of the present invention is to provide a cosmetic composition contained within a capsule whose seal can readily be broken without the aid of a piercing instrument.

A further object of the present invention is to provide a cosmetic composition contained within a capsule whose shape is distinctly different from that of typical vitamin capsules.

A still further object of the present invention is to provide a cosmetic composition contained within a capsule that is generally round yet has means for preventing undesirable roll and has means for being gripped by the fingers.

Other objects, features and advantages of this invention will become more apparent upon reference to the following detailed description and drawings illustrating a preferred embodiment thereof.

SUMMARY OF THE INVENTION

A cosmetic product is provided comprising:
a cosmetic composition pharmaceutically acceptable for application to a human body; and
a capsule completely enclosing the cosmetic composition, the capsule comprising:
(i) a round body with a hollow chamber forming a major portion of the capsule, the cosmetic composition being contained within the chamber;
(ii) a tab forming a minor portion of the capsule; and
(iii) a neck section connecting the tab with the round body, the neck upon being twisted breaking to allow exit of the composition from the chamber.

A means for preventing rolling of the capsule may be provided in the form of an outwardly projecting ring positioned along a median circumference of an outer wall of the round body. Ideally, the capsule should have a Saturn-like appearance wherein the ring functions as the aforesaid roll prevention means.

A variety of substances may be employed to form walls of the capsule. Most preferred as the wall-forming substance is gelatin. Since gelatin is water-soluble, it is important with this embodiment to ensure that the cosmetic product is relatively anhydrous. Among suitable cosmetic compositions are those in lotion, cream or paste form. These products are intended for application to either hair or skin. The skin compositions may include agents providing sunscreen, tanning, anti-wrinkling, anti-dandruff, anti-acne, moisturizing and hair growth benefits.

BRIEF DESCRIPTION OF THE DRAWING

The accompanying drawing will more fully illustrate a selected embodiment of the present invention wherein:

Figure 1 is a top plan view from above showing the capsule;

Figure 2 is a right side elevational view thereof;

Figure 3 is a front side elevational view thereof, similar to Figure 2 but shifted by 90°;

Figure 4 is a rear side elevational view thereof;

Figure 5 is a bottom plan view thereof, corresponding to Figure 1 but rotated by 180°; and

Figure 6 is a left side elevational view thereof.

DETAILED DESCRIPTION

Now it has been discovered that a Saturn-shaped capsule with twist-off handle overcomes many of the disadvantages of the known art.

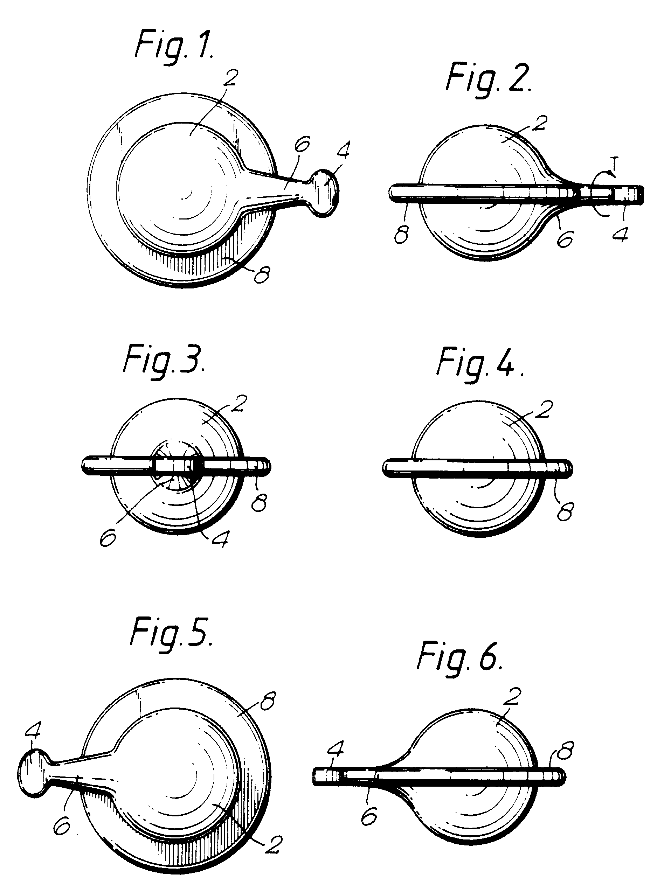

Figures 1-6 illustrate a preferred embodiment of the present invention. The capsule includes a major portion which is a round body having a hollow chamber 2 containing a cosmetic composition pharmaceutically acceptable for application to skin or hair. A tab 4 forms a minor portion of the capsule. This tab preferably may be either round or oblong in shape. To connect tab 4 with the chamber 2, there is provided a neck section 6 which is hollow along at least a partial length thereof. A ring 8 projects outwardly along an equatorial plane of the round body. Rolling away of the capsule is prevented by ring 8.

Tab 4 serves the dual function of a gripping handle and a twist-off opening mechanism. The capsule is easily punctured by twisting in direction T the tab 4 until the neck section 6 snaps thereby causing a passage to open into the chamber. See Figure 2. By gentle squeezing of the capsule walls, cosmetic composition is forced to exit through the puncture opening.

Any cosmetic composition may be employed provided it is pharmaceutically acceptable for application to the human skin or hair. There is the further proviso that the cosmetic composition must also be compatible with the substance that comprises the walls of the capsule.

Capsules of the present invention may be formed from a wide variety of substances which may be of natural or synthetic origin. Most preferred for the present invention is the natural substance commonly known as gelatin.

Gelatin walls may either be soft or hard. According to the present invention, however, the walls are elastic or soft. Gelatin for soft capsules normally will be selected from low-bloom Type A (170-180 g), Type B (150-172 g), or a mixture of Types A and B. The manufacturing process for preparing such capsules can utilize a rotary die fed from two plasticized gelatin sheets which form a sealed chamber or compartment around the material being encapsulated. The size of the capsules may range from No. 0 to 2. Diameter of the combined ring and body may range from 0.5 to 5 cm, preferably 1 to 3 cm, optimally about 1.5 cm. Tab and neck combination will normally be shorter in length than the combined ring and body diameter and will range from 0.1 to 2 cm, preferably 0.3 to 1 cm. Amounts of cosmetic product held within these capsules may range in weight anywhere from 0.05 to 5 grams, preferably from 0.3 to 2 grams, optimally about 1 gram.

A large variety of synthetic polymers may be utilized as the wall-forming substance. The polymers may either be water-soluble or water-insoluble. Suitable materials are polymers derived from such monomers as vinyl chloride, vinyl alcohol, vinyl pyrrolidone, furan, acrylonitrile, vinyl acetate, methyl acrylate, methyl methacrylate, styrene, vinyl ethyl ether, vinyl propyl ether, acrylamide, ethylene, propylene, acrylic acid, methacrylic acid, maleic anhydride, salts of any of the aforementioned acids and mixtures thereof. These materials may be in the form of either homo or copolymers. More specific examples include polyvinyl chloride, polypropylene, acrylic/maleic copolymers, sodium polyacrylate, polyvinyl pyrrolidone and polyvinyl alcohol.

Cellulose based materials may also be suitable; these include sodium carboxymethyl cellulose, hydroxypropyl methyl cellulose, ethyl cellulose, cellulose acetate and cellulose sulphate esters.

Injection molding and extrusion processes are preferable handling procedures when employing synthetic polymers for the present invention. It is also to be understood that plasticizers, protective coatings and other functional additives may be incorporated within the wall material.

Lotion, cream and paste forms may be packaged within the capsule. These compositions may either be anhydrous, aqueous or in emulsion form, the latter encompassing both oil-in-water and water-in-oil emulsions.

Cosmetic compositions of the present invention generally will contain a vehicle or a carrier which is inert, usually an ingredient present in highest amounts, and functioning to deliver active or performance ingredients. The amount of vehicle may range from 5 to 99%, preferably from 25 to 80% by weight of the total composition.

Where the capsule wall material is water sensitive, such as where gelatin, polyvinyl alcohol or polyvinyl pyrrolidone are employed, a nonaqueous carrier becomes necessary. Especially useful in this situation is a silicone polymer, preferably a polydimethyl siloxane and/or a polydimethyl phenyl siloxane. Silicones of this invention may be those with viscosities ranging anywhere from $10^{-5}$ to $10^{-2}$ m$^2$/s (10 to 10,000,000 centistokes) at 25°C. Especially desirable are mixtures of low and high viscosity silicones. These silicones are available from the General Electric Company under the trademarks Vicasil, SE and SF and from the Dow Corning Company under the 200 and 550 Series. Amounts of silicone which can be utilized in the compositions of this invention range anywhere from 5 to 95%, preferably from 25 to 90% by weight of the composition.

Surfactants, which are also sometimes designated as emulsifiers, may be incorporated into the cosmetic compositions of the present invention. Surfactants can comprise anywhere from 0.5 to 30%, preferably from 1 to 15% by weight of the total composition. Surfactants may be cationic, nonionic, anionic, or amphoteric in nature and combinations thereof may be employed.

Illustrative of the nonionic surfactants are alkoxylated compounds based upon fatty alcohols, fatty acids and sorbitan. These materials are available, for instance, from the Shell Chemical Company under the "Neodol" designation. Copolymers of polyoxypropylene-polyoxyethylene, available under the Pluronic® trademark sold by the BASF Corporation, are sometimes also useful. Alkyl polyglycosides available from the Henkel Corporation similarly can be utilized for the purposes of this invention.

Anionic-type surfactants may include fatty acid soaps, sodium lauryl sulphate, sodium lauryl ether sulphate, alkyl benzene sulphonate, mono and dialkyl acid phosphates and sodium fatty acyl isethionate.

Amphoteric surfactants include such materials as dialkylamine oxide and various types of betaines (such as cocoamido propyl betaine).

Emollients are often incorporated into cosmetic compositions of the present invention. Levels of such emollients may range from 0.5 to 50%, preferably between 5 and 30% by weight of the total composition. Emollients may be classified under such general chemical categories as esters, fatty acids and alcohols, polyols and hydrocarbons.

Esters may be mono- or di-esters. Acceptable examples of fatty di-esters include dibutyl adipate, diethyl sebacate, diisopropyl dimerate, and dioctyl succinate. Acceptable branched chain fatty esters include 2-ethylhexyl myristate, isopropyl stearate and isostearyl palmitate. Acceptable tribasic acid esters include triisopropyl trilinoleate and trilauryl citrate. Acceptable straight chain fatty esters include lauryl palmitate, myristyl lactate, oleyl erucate and stearyl oleate. Preferred esters include coco-caprylate/caprate (a blend of coco-caprylate and coco-caprate), propylene glycol myristyl ether acetate, diisopropyl adipate and cetyl octanoate.

Suitable fatty alcohols and acids include those compounds having from 10 to 20 carbon atoms. Especially preferred are such compounds such as cetyl, myristyl, palmitic and stearyl alcohols and acids.

Among the polyols which may serve as emollients are linear and branched chain alkyl polyhydroxyl compounds. For example, propylene glycol, sorbitol and glycerin are preferred. Also useful may be polymeric polyols such as polypropylene glycol and polyethylene glycol.

Exemplary hydrocarbons which may serve as emollients are those having hydrocarbon chains anywhere from 12 to 30 carbon atoms. Specific examples include mineral oil, petroleum jelly, squalene and isoparaffins.

Another category of functional ingredients within the cosmetic compositions of the present invention are thickeners. A thickener will usually be present in amounts anywhere from 0.1 to 20% by weight, preferably from 0.5 to 10% by weight of the composition. Exemplary thickeners are cross-linked polyacrylate materials available under the trademark Carbopol® from the B.F. Goodrich Company. Gums may be employed such as xanthan, carrageenan, gelatin, karaya, pectin and locust beans gum. Under certain circumstances the thickening function may be accomplished by a material also serving as a silicone or emollient. For instance, silicone gums in excess of $10^{-5}$ $m^2/s$ (10 centistokes) and esters such as glycerol stearate have dual functionality.

Various types of active ingredients may be present in cosmetic compositions of the present invention. Actives are defined as skin or hair benefit agents other than emollients and other than ingredients that merely improve the physical characteristics of the composition. Although not limited to this category, general examples include sunscreens, tanning agents, skin anti-wrinkling agents, anti-dandruff agents, anti-acne agents and hair growth stimulants.

Sunscreens include those materials commonly employed to block ultraviolet light. Illustrative compounds are the derivatives of PABA, cinnamate and salicylate. For example, octyl methoxycinnamate and 2-hydroxy-4-methoxy benzophenone (also known as oxybenzone) can be used. Octyl methoxycinnamate and 2-hydroxy-4-methoxy benzophenone are commercially available under the trademarks, Parsol MCX and Benzophenone-3, respectively. The exact amount of sunscreen employed in the emulsions can vary depending upon the degree of protection desired from the sun's UV radiation.

Anti-wrinkling agents are best exemplified by the 2-hydroxyalkanoic acids, prostaglandins, retinoic acids, ceramides and their derivatives. These agents may be present anywhere from 0.00001 to 5%, preferably from 0.0001 to 1%, optimally between 0.01 and 0.2% by weight of the total composition. Most preferred of the active compounds mentioned above is 2-hydroxyoctanoic acid, retinol and pigskin or bovine-brain lipid ceramides. Further identification of ceramide structures may be found in U.S. Patent 4,950,688.

Vitamins may also be included in the compositions of the present invention. Especially preferred is vitamin A palmitate (retinyl palmitate) and vitamin E linoleate (tocopheryl linoleate). Other esters of vitamins A and E may also be utilized.

Many cosmetic compositions, especially those containing water, must be protected against the growth of potentially harmful microorganisms. Preservatives are, therefore, necessary. Suitable preservatives include alkyl esters of p-hydroxybenzoic acid, hydantoin derivatives, propionate salts, and a variety of quaternary ammonium compounds.

Particularly preferred preservatives of this invention are methyl paraben, propyl paraben, imidazolidinyl urea, sodium dehydroxyacetate and benzyl alcohol. Preservatives will usually be employed in amounts ranging from 0.5% to 2% by weight of the composition.

Powders may be incorporated into the cosmetic compositions of the invention. These powders include chalk, talc, Fullers earth, kaolin, starch, smectites clays, chemically modified magnesium aluminum silicate, organically modified montmorillonite clay, hydrated aluminum silicate, fumed silica, aluminum starch octenyl succinate and mixtures thereof.

Other adjunct minor components may also be incorporated into the cosmetic compositions. These ingredients may include coloring agents, opacifiers and perfumes. Amounts of these materials may range anywhere from 0.001 up to 20% by weight of the composition.

The following examples will more fully illustrate selected embodiments of this invention. All parts, percentages and proportions referred to herein and in the appended claims are by weight unless otherwise indicated.

Example 1

A gelatin capsule is prepared having a structure as depicted in the drawing and containing the following cosmetic composition:

| SKINCARE TREATMENT | |
| --- | --- |
| Ingredient | Wt.% |
| Silicone Gum SE-30[1] | 10.00 |
| Silicone Fluid 345[2] | 20.00 |
| Silicone Fluid 344[3] | 58.49 |
| Squalene | 10.00 |
| Ceramides | 0.01 |
| Vitamin A Palmitate | 0.50 |
| Vitamin E Linoleate | 0.50 |
| Herbal Oil | 0.50 |

1. A dimethyl silicone polymer having a molecular weight of at least 50,000 and a viscosity of at least $10^{-2}$ m²/s (10,000 centistokes)at 25°C, available from GEC.
2. Dimethyl siloxane cyclic pentamer, available from Dow Corning Corp.
3. Dimethyl siloxane cyclic tetramer, available from Dow Corning Corp.

Example 2

A polyacrylamide capsule is prepared having a structure as depicted in the drawing and containing the following cosmetic composition:

**SUNTAN LOTION**

| Ingredient | Wt. % |
| --- | --- |
| Water | 86.00 |
| Acetulan (cetyl acetate and acetylated lanolin alcohol) | 4.00 |
| Propylene glycol | 3.00 |
| Stearic acid | 2.00 |
| Dow Corning 556 Fluid (phenyl dimethicone) | 1.00 |
| Veegum (modified magnesium aluminum silicate) | 1.00 |
| Cetyl alcohol | 0.50 |
| Triethanolamine | 0.50 |
| Octyl methoxycinnamate | 1.00 |
| Oxybenzone | 1.00 |
| Preservatives | qs |

Example 3

A cellulose acetate capsule is prepared having a structure as depicted in the drawing and containing the following composition:

### ACNE LOTION

| Ingredient | Wt.% |
|---|---|
| Deionized water | 82.60 |
| Glycerin | 3.00 |
| Glyceryl monstearate | 3.00 |
| Smectite clay | 2.00 |
| Stearyl alcohol | 1.00 |
| Isocetyl stearate | 1.00 |
| Preservatives | 0.40 |
| Benzoyl peroxide | 7.00 |

Example 4

A polypropylene capsule is prepared having a structure as depicted in the drawing and containing the following cosmetic composition:

### SKIN WRINKLE SMOOTHER

| Ingredients | Wt. % |
|---|---|
| Water | 82.50 |
| Flexan 130 (sodiium polystyrene sulfonate) | 12.00 |
| Collasol soluble collagen | 3.00 |
| Modified magnesium aluminum silicate | 1.50 |
| Cellulose gum CMC-7LF | 1.00 |

Example 5

A polyvinyl alcohol capsule is prepared having a structure as depicted in the drawing and containing the following cosmetic composition:

8

### ANTI-DANDRUFF SHAMPOO

| Ingredient | Wt.% |
|---|---|
| Water | 58.55 |
| TEA lauryl sulfate (40%) | 25.00 |
| Hamposyl L-30 fatty acid sarcosinate | 10.00 |
| Zinc pyrithione (48%) | 4.20 |
| Hydroxypropyl methylcellulose | 1.25 |
| Modified magnesium aluminum silicate | 1.00 |

Example 6

A polyvinyl pyrrolidone capsule is prepared having a structure as depicted in the drawing and containing the following cosmetic composition:

### HAIR GROWTH STIMULANT

| Ingredient | Wt.% |
|---|---|
| Water | 60.15 |
| Sodium lauryl ether sulfate | 28.00 |
| Sodium sulfate | 10.00 |
| Lanolin alcohol | 1.00 |
| Polyoxyethylene 20 sorbitan | 0.50 |
| Minoxidil | 0.25 |
| Methylparaben | 0.10 |

The foregoing description and examples illustrate selected embodiments of the present invention and in light thereof variations and modifications will be suggested to one skilled in the art, all of which are within the spirit and purview of this invention.

**Claims**

1. A cosmetic product comprising:
   a cosmetic composition pharmaceutically acceptable for application to a human body; and
   a capsule completely enclosing said cosmetic composition, said capsule having an elastic or soft wall material and comprising:
   (i) a round body with a hollow chamber (2) forming a major portion of said capsule, said cosmetic composition being contained within said chamber (2);
   (ii) a tab (4) forming a minor portion of said capsule; and
   (iii) a neck section (6) connecting said tab (4) with said round body, said neck (6) upon being twisted breaking to allow exit of said composition from said chamber (2),

characterised in that it additionally comprises an outwardly projecting ring (8).

2. A product according to claim 1, wherein said hollow chamber (2) has a capacity for holding from about 0.05 to about 5 grams of said cosmetic composition.

3. A product according to claim 1 or 2, wherein at least a portion of said neck section (6) is hollow.

4. A product according to claim 1, 2 or 3, wherein the outwardly projecting ring (8) circumferentially encompasses an outer wall of said round body.

5. A product according to claim 4, wherein said ring (8) is positioned in a plane equatorially cutting said round body.

6. A product according to any preceding claim, wherein said capsule has walls formed from gelatine.

7. A product according to any preceding claim, wherein said capsule has walls formed of homo and copolymers derived from polymerisation of a monomer chosen from vinyl chloride, vinyl alcohol, vinyl pyrrolidone, furan, acrylonitrile, vinyl acetate, methyl acrylates, methyl methacrylate, styrene, vinyl ethyl ether, vinyl propyl ether, acrylamide, ethylene, propylene, acrylic acid, methacrylic acid, maleic anhydride, salts of any of the aforementioned acids and mixtures thereof.

8. A product according to claim 7, wherein said substance forming said capsule is polyvinyl alcohol.

9. A product according to any of claims 4 to 8, wherein said round body and ring (8) combined have an external diameter ranging from 0.5 to 5 cm.

10. A product according to any preceding claim, wherein said cosmetic composition comprises a vehicle chosen from water, silicone polymer and mixtures thereof.

11. A product according to any preceding claim, wherein said cosmetic composition is substantially anhydrous.

12. A product according to claim 11, wherein said cosmetic composition includes a silicone polymer as a major component thereof.

13. A product according to any preceding claim, wherein said cosmetic composition comprises an active ingredient chosen from sunscreens, tanning agents, skin anti-wrinkle agents, anti-dandruff agents, anti-acne agents, hair growth stimulants and mixtures thereof.

14. A product according to any preceding claim, wherein said cosmetic composition is substantially anhydrous and comprises:
    (i) from 5 to 99% by weight of a nonaqueous carrier comprising silicone polymer; and
    (ii) the balance of the composition comprising effective amounts of other nonaqueous ingredients chosen from emollients, esters, fatty alcohols, thickeners, anti-wrinkle agents, vitamins, oils and mixtures thereof.

15. A product according to claim 14, wherein the nonaqueous carrier comprises from 0.5 to 95% by weight in terms of the total composition of silicone polymer including at least one low viscosity silicone and at least one high viscosity silicone.

16. A product according to claim 15, wherein the low viscosity silicone is chosen from dimethyl siloxane cyclic tetramer, dimethyl siloxane cyclic pentamer and mixtures thereof.

17. A product according to claim 15, wherein the high viscosity silicone is chosen from dimethyl silicone polymers having a molecular weight of at least 50,000 and a viscosity of at least $10^{-2}$ m$^2$/s (10,000 centistokes) at 25°C.

18. A product according to any claims 14 to 17, wherein the nonaqueous ingredients comprises a hydrocarbon emollient chosen from mineral oil, petroleum jelly, squalene, isoparaffins and mixtures thereof.

19. A product according to claims 14 to 18, wherein the nonaqueous ingredient comprises an anti-wrinkle agent chosen from 2-hydroxyalkanoic acids, prostaglandins, retinoic acids, ceramides and their derivatives, and mixtures thereof in an amount of from 0.00001 to 5% by weight of the composition.

20. A product according to any claims 14 to 19, wherein the nonaqueous ingredients comprise vitamin chosen from vitamin A palmitate, vitamin E linoleate and mixtures thereof.

21. A product according to any of claims 14 to 20, which comprises:
(i) about 10% by weight of squalene,
(ii) from 0.001 to 1% by weight ceramide,
(iii) about 1% by weight vitamin chosen from vitamin A palmitate, vitamin E linoleate and fixtures thereof, and
(iv) the balance of the composition comprising silicone gum and silicone fluid in a major amount.

**Patentansprüche**

1. Kosmetisches Produkt, welches umfaßt:
eine kosmetische, zur Anwendung an einem menschlichen Körper pharmazeutisch akzeptable Zusammensetzung; und
eine Kapsel, die die kosmetische Zusammensetzung vollständig enthält, wobei die Kapsel ein elastisches oder weiches Wandmaterial hat und
(i) einen runden Körper mit einer Hohlkammer (2), die einen Hauptteil der Kapsel bildet, wobei die kosmetische Zusammensetzung in der Kammer (2) enthalten ist,
(ii) eine Lasche (4), die einen kleineren Teil der Kapsel bildet, und
(iii) ein Gelenkteil (6), das die Lasche (4) mit dem runden Körper verbindet, wobei das Gelenk (6) bei Drehung bricht und den Austritt der Zusammensetzung aus der Kammer (2) gestattet,
umfaßt, dadurch gekennzeichnet, daß sie zusätzlich einen nach außen ragenden Ring (8) umfaßt.

2. Produkt gemäß Anspruch 1, worin die Hohlkammer (2) ein Fassungsvermögen für ungefähr 0,05 bis ungefähr 5 Gramm der kosmetischen Zusammensetzung hat.

3. Produkt gemäß Anspruch 1 oder 2, worin mindestens ein Teil des Gelenkteils (6) hohl ist.

4. Produkt gemäß Anspruch 1, 2 oder 3, worin der nach außen ragende Ring (8) eine Außenwand des runden Körpers umfangartig umgibt.

5. Produkt gemäß Anspruch 4, worin der Ring (8) in einer Ebene positioniert ist, die den runden Körper äquatorial schneidet.

6. Produkt gemäß irgendeinem vorhergehenden Anspruch, worin die Kapsel Wände hat, die aus Gelatine gebildet werden.

7. Produkt gemäß irgendeinem vorhergehenden Anspruch, worin die Kapsel Wände hat, die aus Homo- und Copolymeren gebildet werden, die aus der Polymerisation eines aus Vinylchlorid, Vinylalkohol, Vinylpyrrolidon, Furan, Acrylnitril, Vinylacetat, Methylacrylaten, Methylmethacrylat, Styrol, Vinylethylether, Vinylpropylether, Acrylamid, Ethylen, Propylen, Acrylsäure, Methacrylsäure, Maleinsäureanhydrid, Salzen irgendeiner der obenerwähnten Säuren und Mischungen davon ausgewählten Monomeren abgeleitet sind.

8. Produkt gemäß Anspruch 7, worin die Substanz, die die Kapsel bildet, Polyvinylalkohol ist.

9. Produkt gemäß irgendeinem der Ansprüche 4 bis 8, worin der runde Körper und Ring (8) zusammen einen äußeren Durchmesser haben, der zwischen 0,5 und 5 cm liegt.

**10.** Produkt gemäß irgendeinem vorhergehenden Anspruch, worin die kosmetische Zusammensetzung ein aus Wasser, Silikonpolymer und Mischungen davon ausgewähltes Transportmedium umfaßt.

**11.** Produkt gemäß irgendeinem vorhergehenden Anspruch, worin die kosmetische Zusammensetzung im wesentlichen wasserfrei ist.

**12.** Produkt gemäß Anspruch 11, worin die kosmetische Zusammensetzung ein Silikonpolymer als eine Hauptkomponente davon einschließt.

**13.** Produkt gemäß irgendeinem vorhergehenden Anspruch, worin die kosmetische Zusammensetzung einen aus Sonnenschutzmitteln, Bräunungsmitteln, Hautantifaltenmitteln, Antischuppenmitteln, Antiaknemitteln, haarwuchsfördernden Stimulantien und Mischungen davon ausgewählten aktiven Inhaltsstoff umfaßt.

**14.** Produkt gemäß irgendeinem vorhergehenden Anspruch, worin die kosmetische Zusammensetzung im wesentlichen wasserfrei ist und umfaßt:
(i) zwischen 5 und 99 Gew.-% eines nichtwäßrigen Trägerstoffs, der Silikonpolymer umfaßt, und
(ii) wobei der Rest der Zusammensetzung effektive Mengen anderer nichtwäßriger aus weichmachenden Mitteln, Estern, Fettalkoholen, Verdickungsmitteln, Antifaltenmitteln, Vitaminen, Ölen und Mischungen davon ausgewählte Inhaltsstoffe umfaßt.

**15.** Produkt gemäß Anspruch 14, worin der nichtwäßrige Trägerstoff zwischen 0,5 und 95 Gew.-% der gesamten Silikonpolymerzusammensetzung, die mindestens ein niedrigviskoses Silikon und mindestens ein hochviskoses Silikon einschließt, umfaßt.

**16.** Produkt gemäß Anspruch 15, worin das niedrigviskose Silikon aus cyclischem Dimethylsiloxan-Tetramer, cyclischem Dimethylsiloxan-Pentamer und Mischungen davon ausgewählt wird.

**17.** Produkt gemäß Anspruch 15, worin das hochviskose Silikon aus Dimethylsilikonpolymeren ausgewählt wird, die ein Molekulargewicht von mindestens 50.000 und bei 25 °C eine Viskosität von mindestens $10^{-2}$ m²/s (10.000 Centistokes) haben.

**18.** Produkt gemäß irgendeinem der Ansprüche 14 bis 17, worin die nichtwäßrigen Inhaltsstoffe ein aus Mineralöl, Petrolat, Squalen, Isoparaffinen und Mischungen davon ausgewähltes erweichendes Mittel auf Kohlenwasserstoffbasis umfassen.

**19.** Produkt gemäß ,Ansprüchen 14 bis 18, worin der nichtwäßrige Inhaltsstoff ein aus 2-Hydroxyalkansäuren, Prostaglandinen, Retinolsäuren, Ceramiden und deren Derivaten und Mischungen davon ausgewähltes Antifaltenmittel in einer Menge von 0,00001 bis 5 Gew.-% der Zusammensetzung umfaßt.

**20.** Produkt gemäß irgendeinem der Ansprüche 14 bis 19, worin die nichtwäßrigen Inhaltsstoffe ein aus Vitamin-A-Palmitat, Vitamin-E-Linoleat und Mischungen davon ausgewähltes Vitamin umfassen.

**21.** Produkt gemäß irgendeinem der Ansprüche 14 bis 20, welches umfaßt:
(i) ungefähr 10 Gew.-% Squalen,
(ii) zwischen 0,001 und 1 Gew.-% Ceramid,
(iii) ungefähr 1 Gew.-% aus Vitamin-A-Palmitat, Vitamin-E-Linoleat und Mischungen davon ausgewähltes Vitamin, und
(iv) wobei der Rest der Zusammensetzung Silikongummi und Silikonöl in einer größeren Menge umfaßt.

**Revendications**

**1.** Produit cosmétique comprenant :
- une composition cosmétique pharmaceutiquement acceptable pour application au corps humain ; et
- une capsule entourant complètement ladite composition cosmétique, ladite capsule présentant une matière de parois élastique ou molle et comprenant :

(i) un corps rond ayant une chambre creuse (2) qui forme la portion principale de ladite capsule, ladite composition cosmétique étant contenue dans ladite chambre (2);

(ii) une languette (4) qui constitue une portion secondaire de ladite capsule ; et

(iii) un col (6) reliant ladite languette (4) avec ledit corps rond, ledit col (6), quand il est tordu se brise pour permettre la sortie de ladite composition de ladite chambre (2),

caractérisé en ce qu'il comprend en outre une bague (8) faisant saillie vers l'extérieur.

2. Produit selon la revendication 1, dans lequel ladite chambre creuse (2) possède une capacité lui permettant de contenir environ 0,05 à 5 g de ladite composition cosmétique.

3. Produit selon la revendication 1 ou 2, dans lequel une portion au moins dudit col (6) est creuse.

4. Produit selon la revendication 1, 2 ou 3 dans lequel la bague (8) faisant saillie vers l'extérieur entoure circonférentiellement une paroi extérieure dudit corps rond.

5. Produit selon la revendication 4, dans lequel ladite bague (8) est disposée dans un plan intersectant de façon équatoriale ledit corps rond.

6. Produit selon l'une quelconque des revendications précédentes, dans lequel ladite capsule présente des parois en gélatine.

7. Produit selon l'une quelconque des revendications précédentes, dans lequel ladite capsule présente des parois en homo- et copolymères provenant de la polymérisation d'un monomère choisi parmi le chlorure de vinyle, l'alcool vinylique, la vinylpyrrolidone, le furanne, l'acrylonitrile, l'acétate de vinyle, les acrylates de méthyle, le méthacrylate de méthyle, le styrène, l'éther vinyléthylique, l'éther vinylpropylique, l'acrylamide, l'éthylène, le propylène, l'acide acrylique, l'acide méthacrylique, l'anhydride maléique, les sels de tous les acides précités et leurs mélanges.

8. Produit selon la revendication 7, dans lequel ladite substance formant ladite capsule est l'alcool polyvinylique.

9. Produit selon l'une quelconque des revendications 4 à 8, dans lequel ledit corps rond et la bague (8) combinés ont un diamètre extérieur compris entre 0,5 et 5 cm.

10. Produit selon l'une quelconque des revendications précédentes. dans lequel ladite composition cosmétique comprend un véhicule qui est l'eau, un polymère siliconique ou un mélange de ceux-ci.

11. Produit selon l'une quelconque des revendications précédentes, dans lequel ladite composition cosmétique est en grande partie anhydre.

12. Produit selon la revendication 11, dans lequel ladite composition cosmétique comprend un polymère siliconique à titre d'un composant principal.

13. Produit selon l'une quelconque des revendications précédentes, dans lequel ladite composition cosmétique comprend un ingrédient actif choisi parmi les filtres solaires, les agents de bronzage, les agents anti-rides, les agents anti-pelliculaires, les agents anti-acné, les stimulants de la croissance capillaire et les mélanges de ceux-ci.

14. Produit selon l'une quelconque des revendications précédentes, dans lequel ladite composition cosmétique est sensiblement anhydre et comprend :

(i) de 5 à 99 % en poids d'un véhicule non aqueux comprenant un polymère siliconique ; et

(ii) le complément de la composition comprenant des proportions efficaces d'autres ingrédients non aqueux choisis parmi les émollients, les esters, les alcools gras, les épaississants, les agents anti-rides, les vitamines, les huiles et leurs mélanges.

15. Produit selon la revendication 14, dans lequel le véhicule non aqueux comprend de 0,5 à 95 % en poids par rapport à la composition totale, d'un polymère siliconique comprenant au moins une silicone de basse viscosité et au moins une silicone de haute viscosité.

**16.** Produit selon la revendication 15, dans lequel la silicone de basse viscosité est choisie parmi un tétramère cyclique de diméthylsiloxane, un pentamère cyclique de diméthylsiloxane et leurs mélanges.

**17.** Produit selon la revendication 15, dans lequel la silicone de haute viscosité est choisie parmi les polymères de diméthylsilicone ayant une masse moléculaire d'au moins 50.000 et une viscosité d'au moins $10^{-2}$ m$^2$/s (10.000 cs) à 25°C.

**18.** Produit selon l'une quelconque des revendications 14 à 17, dis lequel les ingrédients non aqueux comprennent un émollient hydrocarboné choisi parmi l'huile minérale, la gelée de pétrole, le squalène, les isoparaffines et leurs mélanges.

**19.** Produit selon la revendication 14 à 18, dans lequel l'ingrédient non aqueux comprend un agent anti-rides choisi parmi les acides 2-hydroxyalcanoïques, les prostaglandines, les acides rétinoïques, les céramides, et leurs dérivés ainsi que leurs mélanges à raison de 0,00001 à 5 % en poids de la composition.

**20.** Produit selon l'une quelconque des revendications 14 à 19, dans lequel les ingrédients non aqueux comprennent une vitamine choisie parmi le palmitate de vitamine A, le linoléate de vitamine E et leurs mélanges.

**21.** Produit selon l'une quelconque des revendications 14 à 20, qui comprend :
(i) environ 10 % en poids de squalène,
(ii) de 0,001 à 1 % en poids de céramide,
(iii) environ 1 % en poids d'une vitamine choisie parmi le palmitate de vitamine A, le linoléate de vitamine E et leurs mélanges, et
(iv) le complément de la composition comprenant une gomme siliconique et un fluide siliconique en une proportion principale.

Fig. 1.

Fig. 2.

Fig. 3.

Fig. 4.

Fig. 5.

Fig. 6.